# DEMANDE DE BREVET EUROPEEN

(11) **EP 3 597 137 A1**
(43) Date de publication de la demande: **22.01.2020**
(21) Numéro de dépôt: 19196022.8
(22) Date de dépôt: 19.04.2012
(51) Int. Cl.: A61B 50/30, A61C 8/00, A61F 2/00, A61B 17/86, A61L 2/26

(54) **ENSEMBLE MEDICAL COMPRENANT UN OBJET MEDICAL ET UN EMBALLAGE CONTENANT LEDIT OBJET**

(30) Priorité: 22.04.2011 FR 1153520
(62) Demande divisionnaire de: 12722437.6
(71) Demandeur: Synthes GmbH, 4436 Oberdorf (CH)
(72) Inventeur: RICHART, Olivier, 17140 Lagord (FR); LARCHE, Grégoire, 49300 Cholet (FR); PODGORSKI, Jean-Pierre, 49230 Saint Crespin Sur Moine (FR)
(74) Mandataire: Klunker IP Patentanwälte PartG mbB

(57) **Abrégé**

L'invention concerne un ensemble médical, de préférence stérilisé, comprenant un objet (5) médical et un emballage contenant ledit objet, ledit emballage comprenant un élément support (2) creux à l'intérieur duquel ledit objet (5) est libre, un premier élément de protection (3), appelé bouchon, couplable à l'élément support (2) dudit objet, de telle sorte que l'élément support (2) présente une partie (20) de saisie qui s'étend en saillie dudit bouchon (3), et un deuxième élément de protection (4) creux, appelé coiffe. Ladite coiffe est couplable au bouchon (3) de manière à délimiter, en coopération avec ledit bouchon (3), une chambre (8) à l'intérieur de laquelle s'étend ladite partie de saisie (20) de l'élément support, et, à l'état couplé de la coiffe au bouchon, une partie du bouchon (3) s'étend en saillie de la coiffe (4).

## Description

La présente invention concerne de manière générale les emballages pour objets, en particulier pour pièces médicales de préférence stérilisées.

L'invention concerne plus particulièrement un ensemble médical, de préférence stérilisé, comprenant un objet médical et un emballage contenant ledit objet.

On connaît de l'état de la technique des emballages se présentant sous la forme de double enveloppes externe et interne de type sachets ou double coques plastiques blister ou d'une combinaison des deux dans lesquels un objet est contenu. Dans le cas de pièces médicales devant être déconditionnées dans des conditions proches de l'asepsie, de tels emballages peuvent poser des problèmes de contamination lors du passage de l'emballage d'une personne à une autre et de l'ouverture dudit emballage. En effet, depuis son départ d'une zone de travail dite sale, c'est-à-dire sans condition d'asepsie particulière, jusqu'à son arrivée dans une zone de travail dite propre, c'est-à-dire une zone de travail dans laquelle des conditions d'asepsie données sont respectées, par exemple la zone « stérile » du bloc opératoire, l'emballage qui arrive en zone propre est contaminé au niveau de son enveloppe extérieure, ce qui présente un risque de contamination de la pièce médicale en sortant celle-ci de l'emballage.

Pour limiter le risque de contamination de l'objet contenu dans l'emballage, il est connu d'ouvrir ledit emballage de manière à faire tomber l'objet sur un plan de travail pour ne pas avoir à toucher ledit objet. Seul le chirurgien ou l'assistant stérile prend l'objet. Cependant, l'objet risque non seulement de se détériorer sous le choc avec le plan de travail, dans le cas d'un objet solide, ou encore de tomber par terre en roulant, mais également d'être contaminé par des corps étrangers présents sur le plan de travail. Il arrive également que l'opérateur tende l'enveloppe extérieure de l'emballage à l'assistant stérile qui doit alors essayer de prendre au mieux l'enveloppe interne sans commettre de faute d'asepsie. Cependant, le risque que l'assistant stérile touche une zone contaminée de l'emballage au cours de cette opération et que l'objet saisi soit à son tour contaminé est très important.

Le document US5062800 décrit un emballage médical pour implant dentaire qui comprend un élément support muni de moyens de fixation de l'implant et un bouchon couplable à l'élément support de telle sorte que l'élément support s'étend en saillie dudit bouchon. Ledit emballage comprend également une coiffe qui recouvre le bouchon. Cependant, la coiffe recouvre entièrement la longueur du bouchon de sorte que l'opérateur n'a pas d'autre solution que de vider le contenu de la coiffe en le faisant tomber sur un plan de travail. Comme rappelé ci-dessus, le fait de faire tomber l'implant, éventuellement avec son enveloppe, peut détériorer ledit implant et augmente le risque de contamination.

En outre, dans le document US5062800, l'implant est fixé à l'élément support correspondant de l'emballage. Il en résulte que l'opérateur doit réaliser une manipulation spécifique pour extraire l'implant de l'élément support une fois les autres éléments retirés, ce qui complique l'intervention de l'opérateur et ne lui permet pas de saisir rapidement l'implant dont il a besoin. En particulier, dans le domaine urgentiste ou de la traumatologie, l'opérateur qui a besoin dudit implant doit pouvoir en disposer le plus rapidement possible, tout en limitant bien entendu le risque de contamination dudit implant.

La présente invention a pour but de proposer un emballage pour le conditionnement et le déconditionnement d'objet qui permet de limiter les risques de contamination de l'objet tout en permettant un accès aisé et rapide audit objet.

A cet effet, l'invention a pour objet un ensemble médical, de préférence stérilisé, comprenant un objet médical et un emballage contenant ledit objet, ledit emballage comprenant un élément support creux à l'intérieur duquel ledit objet est libre,
caractérisé en ce que ledit emballage comprend :
- un premier élément de protection, appelé bouchon, couplable à l'élément support dudit objet, de préférence par emboitement à recouvrement partiel, de telle sorte que l'élément support présente une partie de saisie qui s'étend en saillie dudit bouchon,
ledit élément support délimitant à lui seul ou en coopération avec le bouchon une première chambre renfermant ledit objet,
- un deuxième élément de protection creux, appelé coiffe,
ladite coiffe étant couplable au bouchon, de préférence par emboitement à recouvrement partiel, de manière à délimiter en coopération avec ledit bouchon une deuxième chambre à l'intérieur de laquelle s'étend la partie de saisie de l'élément support,
et en ce que, à l'état couplé de la coiffe au bouchon, une partie du bouchon s'étend en saillie de la coiffe.

Grâce à la partie de saisie de chaque élément de protection et de l'élément support, qui s'étend en saillie de l'élément de protection avec lequel il est couplé, les chambres définies par emboitement à recouvrement partiel desdits éléments peuvent être ouvertes jusqu'à accéder à l'objet sans jamais lâcher ou toucher ledit objet et avec un risque réduit de contamination de l'objet.

En effet lors du passage de l'emballage d'une première personne dont les mains sont supposées "contaminées" à une deuxième personne dont les mains sont supposées "propres", la coiffe de protection peut être retirée par la première personne de manière à ouvrir la "deuxième" chambre pour découvrir l'extrémité de saisie de l'élément support de l'objet et ne conserver pour la deuxième personne que le sous-ensemble formé du bouchon et de l'élément support.

La première personne tient ledit sous-ensemble par l'extrémité du bouchon opposée à l'élément support et peut tendre l'extrémité propre de l'élément support, découverte par l'ouverture de la première chambre, à la deuxième personne, par exemple le chirurgien.

Le bouchon et l'élément support peuvent alors être séparés de manière à ouvrir la chambre contenant l'objet. La deuxième personne, qui ne conserve ainsi que l'élément support qui contient l'objet, peut alors de sa main propre qui tient l'extrémité de saisie de l'élément support, saisir l'objet par exemple par son autre main propre.

Une telle conception de l'emballage selon l'invention permet de réaliser un emballage avec un nombre de pièces réduit, principalement trois, de telle sorte que, lors du passage de l'emballage depuis une zone sale jusqu'à une zone propre par l'intermédiaire de différentes personnes, ni l'élément support ni l'objet ne sont touchés par une personne en zone sale et l'objet contenu dans l'élément support n'est jamais lâché. Le risque de contamination est ainsi très fortement réduit.

En outre, le fait que l'objet soit libre à l'intérieur de l'élément support permet un accès aisé et rapide audit objet. En particulier, dans le domaine urgentiste, la personne qui a besoin dudit objet peut disposer rapidement dudit objet, tout en limitant le risque de contamination dudit objet comme détaillé ci-après.

Selon une caractéristique avantageuse de l'invention, ledit bouchon comprend une paroi périphérique dite interne, et une paroi périphérique dite externe qui entoure à écartement ladite paroi périphérique interne pour définir entre lesdites parois périphériques un espace annulaire d'emboitement de la partie de la coiffe destinée à être couplée audit bouchon, la paroi périphérique interne définissant un espace d'emboitement de la partie de l'élément support destinée à être couplée audit bouchon.

Selon une caractéristique avantageuse de l'invention, la face interne de la paroi périphérique interne du bouchon est munie de moyens de maintien permettant de maintenir la partie de l'élément support emboitée à l'intérieur de l'espace délimité par ladite paroi périphérique interne du bouchon.

Selon une caractéristique avantageuse de l'invention, lesdits moyens de maintien comprennent des ergots qui sont destinés à être en contact d'appui avec l'élément support à l'état emboité dudit élément support dans le bouchon, et les ergots sont répartis sur la face périphérique interne du bouchon, autour de l'axe dudit bouchon, en étant espacés angulairement les uns des autres.

Selon une caractéristique avantageuse de l'invention, chaque ergot comprend une partie formant rampe, dirigée vers l'axe du bouchon en partant d'un point bas de la rampe situé du côté de l'extrémité ouverte du bouchon vers un point haut de ladite rampe situé du côté de l'extrémité fermée dudit bouchon, de sorte que, lors de l'emboitement de l'élément support à l'intérieur de l'espace défini par la paroi périphérique interne du bouchon, ledit élément support, par appui sur ladite rampe, pousse radialement ledit ergot et déforme la paroi périphérique interne correspondante vers la paroi périphérique externe.

Selon une caractéristique avantageuse de l'invention, les ergots forment des entretoises permettant de maintenir un écartement radial entre l'élément support et la paroi périphérique interne du bouchon, l'espacement entre les ergots définissant un espace de communication de fluide entre l'intérieur de la paroi périphérique du bouchon et l'intérieur de la coiffe.

Selon une caractéristique avantageuse de l'invention, lesdites parois périphériques externe et/ou interne du bouchon comprennent des moyens de vissage aptes à coopérer avec des moyens de vissage complémentaires ménagés sur la coiffe.

Selon une caractéristique avantageuse de l'invention, la face interne de la paroi périphérique du bouchon externe présente un taraudage apte à coopérer avec un filet ménagé sur la face externe de la paroi périphérique de la coiffe.

Selon une caractéristique avantageuse de l'invention, ledit élément support délimitant à lui seul ladite première chambre renfermant ledit objet, ledit élément support est formé d'au moins deux pièces couplables/désaccouplables l'une par rapport à l'autre, de préférence par emboitement à recouvrement partiel, pour former ladite première chambre renfermant ledit objet.

Ainsi, la conception de l'emballage sous la forme d'un élément support couplé à un bouchon qui délimite en coopération avec une coiffe une chambre dans laquelle une partie de l'élément support s'étend en saillie du bouchon, permet de faire passer l'élément support d'une première personne à une deuxième personne sans que la première personne ne touche l'élément support et avec un risque réduit de contamination entre la première et la deuxième personne, de sorte que l'élément support qui renferme ledit objet arrive en zone propre avec un risque réduit de contamination. En outre, la conception de l'élément support sous la forme de deux pièces, couplables pour délimiter une cavité fermée (première chambre), permet de protéger ledit objet en attente d'util isation.

Selon une caractéristique avantageuse de l'invention, ledit élément support délimitant en coopération avec le bouchon ladite première chambre renfermant ledit objet, ledit élément support délimite une cavité ouverte destinée à être refermée par ledit bouchon.

Selon un tel mode de réalisation, ledit emballage peut être formé de seulement trois pièces, à savoir la coiffe, le bouchon, et l'élément support. Chaque pièce se présente sous la forme d'un corps creux ouvert à la manière d'une éprouvette. Ainsi, l'élément support est couplable au bouchon pour délimiter la première chambre qui renferme ledit objet et le bouchon est couplable à la coiffe pour délimiter la deuxième chambre qui renferme la partie de l'élément support qui s'étend en saillie du bouchon.

Selon une caractéristique avantageuse de l'invention, le bouchon et la coiffe sont chacun formés par un corps creux allongé ouvert à une extrémité et fermé à l'autre extrémité.

Le caractère allongé de l'un ou des éléments de protection de l'objet formés par la coiffe et le bouchon permet de bénéficier d'une distance de sécurité lors de l'ouverture des chambres au cours du passage des différentes parties de l'emballage d'une personne à une autre.

Selon une caractéristique avantageuse de l'invention, ledit bouchon, et éventuellement la coiffe, présente(nt) des emplacements prédéfinis permettant de positionner au moins deux doigts, de préférence trois doigts, d'une personne pour lui permettre de saisir ledit bouchon, et éventuellement ladite coiffe, au niveau desdits emplacements prédéfinis.

Avantageusement, ledit bouchon, et éventuellement ladite coiffe, présente(nt) des emplacements prédéfinis permettant de positionner au moins trois doigts pour saisir par pincement ledit bouchon, et éventuellement ladite coiffe.

Un tel emplacement prédéfini permet de limiter le risque de contamination de l'élément support et donc de l'objet contenu dans ledit élément support.

Selon une caractéristique avantageuse de l'invention, ladite coiffe étant couplable au bouchon par emboitement à recouvrement partiel, ledit bouchon présente au moins une partie, de préférence au moins une ailette, déformable configurée pour, d'une part, à l'état recouvert de ladite partie déformable du bouchon par la coiffe, venir en appui contre la face interne de coiffe afin de permettre un couplage du bouchon à la coiffe par friction et, d'autre part, pour pouvoir se déformer afin de permettre la séparation de la coiffe du bouchon.

Une telle partie déformable du bouchon assure une bonne étanchéité entre le bouchon et la coiffe à l'état couplé de ces deux pièces.

Selon une caractéristique avantageuse de l'invention, lesdits éléments de support et de protection de l'emballage s'emboitent à recouvrement partiel entre eux par friction et/ou par vissage.

Selon une caractéristique avantageuse de l'invention, l'élément support et/ou le bouchon et/ou la coiffe présente(nt) une partie, de préférence formée par une membrane, imperméable aux bactéries mais perméable aux gaz pour permettre une stérilisation gazeuse de ladite première chambre et/ou de ladite deuxième chambre.

L'invention concerne également un procédé de déballage d'un objet médical contenu dans un emballage d'ensemble médical, de préférence stérilisé, tel que décrit ci-dessus, caractérisé en ce que ledit procédé comprend les étapes suivantes :
a) - séparation de la coiffe par rapport au bouchon par une première personne, dite personne contaminée, de manière à découvrir l'élément support,
b) - saisie par une deuxième personne, dite personne non contaminée, de l'élément support,
c) - séparation dudit élément support par rapport au bouchon tenu par ladite personne contaminée.

Selon une caractéristique avantageuse de l'invention, ledit procédé comprend les étapes supplémentaires suivantes :
d) - éventuellement, mise en attente dudit élément support qui renferme l'objet dans une zone d'attente, en attente de l'utilisation dudit objet,
e) - ouverture de la première chambre définie par ledit élément support pour en extraire ledit objet.

Ces étapes de procédé s'appliquent en particulier à un mode de réalisation de l'emballage selon l'invention, pour lequel l'élément support forme à lui seul ladite première chambre et pour lequel ledit élément support est formé d'au moins deux pièces couplables/désaccouplables l'une à l'autre qui, à l'état couplé, délimitent une cavité fermée formant ladite première chambre. La première personne tend l'emballage restant formé du bouchon et de l'élément support à la deuxième personne en lui présentant l'extrémité dudit emballage restant formée par ledit élément support. La deuxième personne tire alors sur ledit élément support de manière à le séparer dudit bouchon. L'élément support qui forme une cavité qui renferme l'objet peut être posé sur une table dans une zone dite décontaminée en attendant d'être utilisé. La réalisation de l'élément support sous forme d'une cavité qui renferme l'objet permet de protéger ledit objet qui reste, à l'intérieur dudit élément support, en attente d'utilisation contre tout risque de contamination extérieure. Il suffit alors à la deuxième personne, ou à une autre personne "propre" d'ouvrir la première chambre formée par ledit élément support en séparant au moins les deux pièces qui forment ledit élément support.

Selon une caractéristique avantageuse de l'invention, ledit procédé comprend entre les étapes a) et b) l'étape supplémentaire suivante de présentation, par la première personne à la deuxième personne, de l'emballage restant, formé du bouchon et de l'élément support, en lui présentant l'extrémité dudit emballage restant formée par ledit élément support, en orientant ledit emballage restant de telle sorte que l'élément support se retrouve à une hauteur inférieure à celle du bouchon de sorte que ledit objet reste contenu dans l'élément support lors de la séparation dudit élément support par rapport au bouchon pour éviter que ledit objet ne tombe par terre.

Cette étape du procédé s'applique en particulier à un mode de réalisation de l'emballage selon l'invention pour lequel l'élément support forme ladite première chambre en coopération avec le bouchon et ledit élément support se présente sous la forme d'un corps creux allongé, ouvert à une extrémité et fermé à l'autre extrémité, apte à contenir ledit objet. La première personne dite "contaminée" tend l'emballage restant formé du bouchon et de l'élément support à la deuxième personne dite "propre" ou "stérile" en lui présentant l'extrémité dudit emballage restant formée par ledit élément support, en orientant ledit emballage restant de telle sorte que l'élément support se retrouve à une hauteur inférieure à celle du bouchon de sorte que ledit objet reste contenu dans l'élément support lors de la séparation dudit élément support par rapport au bouchon pour éviter que ledit objet ne tombe par terre. La deuxième personne, ou une autre personne propre, peut alors saisir, éventuellement à l'aide d'un outil, l'objet en contact avec ledit élément support.

L'invention sera bien comprise à la lecture de la description suivante d'exemples de réalisation, en référence aux dessins annexés dans lesquels :
- la figure 1 est une vue d'un emballage selon l'invention conformément à un premier mode de réalisation, à l'intérieur duquel est conditionnée une pièce médicale ;
- les figures 2 à 4 sont des vues de l'emballage de la figure 1 au cours d'une première étape de déconditionnement pour laquelle la coiffe est retirée par une première personne dite personne contaminée, le sous-ensemble restant formé du bouchon et du support de pièce étant maintenu côté bouchon par la main de la personne contaminée ;
- les figures 5 et 6 sont des vues du sous-ensemble de l'emballage de la figure 4 au cours d'une deuxième étape de déconditionnement pour laquelle le bouchon est retiré en restant dans la main de la personne contaminée, et l'élément support de l'objet est tenu par la main d'une deuxième personne dite personne propre ;
- la figure 7 est une vue de l'élément support de la figure 5 au cours d'une troisième étape de déconditionnement pour laquelle la personne propre sépare les deux pièces qui forment l'élément support pour en extraire la pièce médicale ;
- la figure 8 est une vue en perspective du bouchon de l'emballage conforme au premier mode de réalisation de l'invention;
- la figure 8A est une vue en perspective d'une variante de réalisation du bouchon de l'emballage selon l'invention avec une zone de positionnement de doigts dite externe ménagée sur la partie du bouchon destinée à s'étendre en saillie de la coiffe;
- la figure 8B est une vue en perspective d'une autre variante de réalisation du bouchon de l'emballage selon l'invention avec une zone de positionnement de doigts dite interne ménagée sur la partie du bouchon destinée à s'étendre à l'intérieur de la coiffe.

On entend par personne "sale" ou "contaminée" une personne travaillant dans des conditions non aseptisées, qui est susceptible de contaminer par ses mains les objets qu'elle touche. A l'inverse, on entend par personne "propre" une personne travaillant dans des conditions d'asepsie suffisante.

En référence aux figures et comme rappelé ci-dessus, l'invention concerne un ensemble médical comprenant un objet 5 médical et un emballage 1 contenant ledit objet 5 à des fins de préservation du caractère stérile de l'objet et en vue du déballage dudit objet dans des conditions aseptiques ou proches de l'asepsie. Ledit objet peut être par exemple une pièce solide, telle qu'une vis pour chirurgie, ou tout autre type d'objet, en particulier tout autre type d'implant. En outre, ledit objet peut être un liquide ou une poudre. Ledit objet et, de préférence, les différentes parties de l'emballage, sont stérilisés comme détaillé ci-après.

Ledit emballage comprend un élément support 2 creux à l'intérieur duquel ledit objet 5 est libre. Autrement dit, ledit objet 5 peut se déplacer librement à l'intérieur de l'élément support 2. Ledit emballage comprend également un premier élément de protection 3, appelé bouchon, couplable à l'élément support 2 dudit objet 5 par emboitement à recouvrement partiel, de telle sorte que l'élément support 2 présente une partie 20 de saisie qui s'étend en saillie dudit bouchon 3. Ladite partie 20 de saisie correspond à la partie d'extrémité libre de l'élément support 2 qui s'étend en saillie dudit bouchon 3. Comme détaillé ci-après, ledit élément support 2 délimite à lui seul ou en coopération avec le bouchon 3 une première chambre 7 renfermant ledit objet 5.

Ledit emballage comprend également un deuxième élément de protection 4 creux, appelé coiffe. Ladite coiffe 4 est couplable au bouchon 3, par emboitement à recouvrement partiel, de manière à délimiter en coopération avec ledit bouchon 3 une deuxième chambre 8 à l'intérieur de laquelle s'étend l'extrémité de saisie 20 de l'élément support 2. A l'état couplé de la coiffe 4 au bouchon 3, une partie 30 du bouchon 3 s'étend en saillie de la coiffe 4.

Ladite partie 30 du bouchon correspond à l'extrémité libre du bouchon 3, opposée à l'extrémité 31 dudit bouchon 3 qui recouvre une partie de l'élément support 2 et forme une zone de saisie. L'extrémité 40 de la coiffe 4 opposée au bouchon 3 forme également une partie de saisie de l'emballage opposée à l'extrémité 30 du bouchon.

Ainsi, chaque élément de l'emballage, à savoir l'élément support 2, le bouchon 3 et la coiffe 4, présente une extrémité de saisie 20, 30, 40 par rapport à son extrémité opposée couplée avec un autre élément de l'emballage. Lesdits éléments s'emboitent entre eux de manière à définir, par coopération de deux éléments emboités, une chambre 7, 8 de préférence étanche au moins aux bactéries. Ainsi, les éléments emboités de l'emballage définissent deux chambres 7, 8 dont une 7, définie entre le bouchon et la coiffe, à l'intérieur de laquelle est logé l'objet et à laquelle on ne peut accéder qu'une fois ouverte l'autre chambre 8 servant de protection de l'extrémité de saisie 20 de l'élément support 2.

Une telle conception de l'emballage permet de le manipuler sans risque de contamination de l'objet contenu dans la chambre de présentation, sans avoir à lâcher ledit objet contenu dans l'élément support, tout en permettant de saisir rapidement ledit objet puisque celui-ci est libre dans l'élément support.

Dans l'exemple illustré aux figures, ledit élément support 2 délimite à lui seul ladite première chambre 7 renfermant ledit objet 5. A cet effet, ledit élément support 2 est formé d'au moins deux pièces 20, 21 couplables/ désaccouplables l'une par rapport à l'autre, par emboitement à recouvrement partiel, pour former ladite première chambre renfermant ledit objet 5.

On peut également prévoir un mode de réalisation selon lequel ledit élément support 2 délimite à lui seul ladite première chambre 7 en étant formé de manière monobloc. Ledit élément support 2 peut ainsi se présenter sous la forme d'un corps creux fermé monobloc sécable, tel qu'une ampoule sécable, contenant ledit objet. Dans ce cas, l'ouverture de la chambre 7 s'effectue en cassant ladite ampoule.

Selon une variante de réalisation non illustrée, ledit élément support délimite en coopération avec le bouchon ladite première chambre renfermant ledit objet. Dans ce cas, ledit élément support délimite une cavité ouverte destinée à être refermée par ledit bouchon.

Préférentiellement, le bouchon 3 est formé par un corps creux allongé ouvert à une extrémité et fermé à l'autre extrémité. Dans l'exemple illustré aux figures, la coiffe 4 est également formée par un corps creux allongé ouvert à une extrémité et fermé à l'autre extrémité. L'élément support 2 se présente également sous la forme d'un corps creux allongé fermé à une extrémité et ouverte à l'autre extrémité, ou fermé à ses deux extrémités et formé d'au moins deux pièces, suivant le mode de réalisation choisi.

La conception du bouchon 3 et de la coiffe 4 sous forme d'un corps allongé à la manière d'une éprouvette ou d'un tube fermé à au moins une extrémité, permet de bénéficier, d'une part, d'une distance de sécurité entre l'extrémité de saisie 40 de la coiffe 4 et l'extrémité de saisie 30 du bouchon 3 et, d'autre part, d'une distance de sécurité entre l'extrémité de saisie 20 de l'élément support 2 et l'extrémité de saisie 30 du bouchon 3.

Une telle distance entre les extrémités de saisie 30, 40 de l'emballage permet de limiter le risque de contamination au niveau de l'extrémité de saisie 20 de l'élément support 2 qui s'étend dans la chambre 8, lorsque ladite chambre 8 est ouverte par séparation de la coiffe 4 et du bouchon 3.

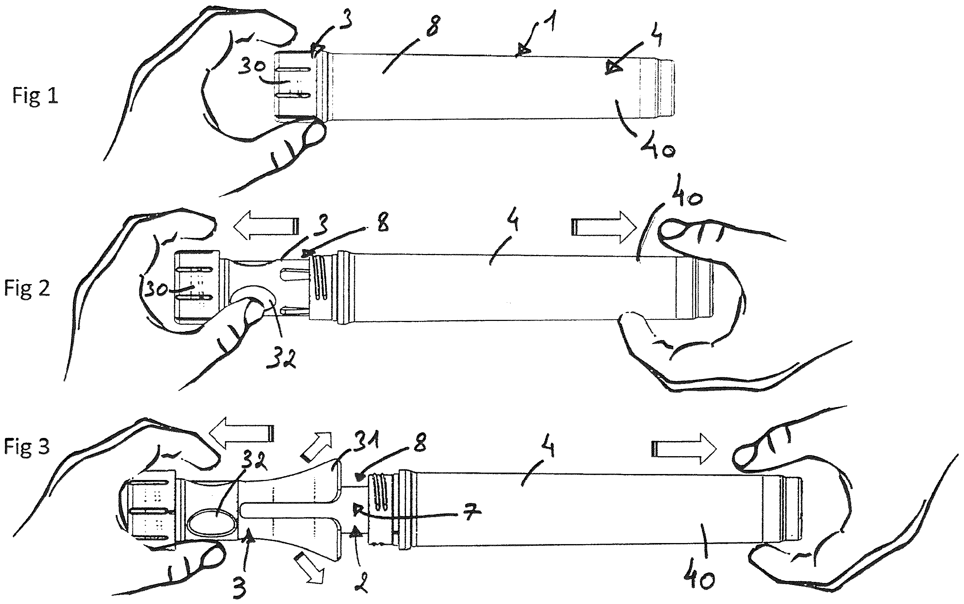

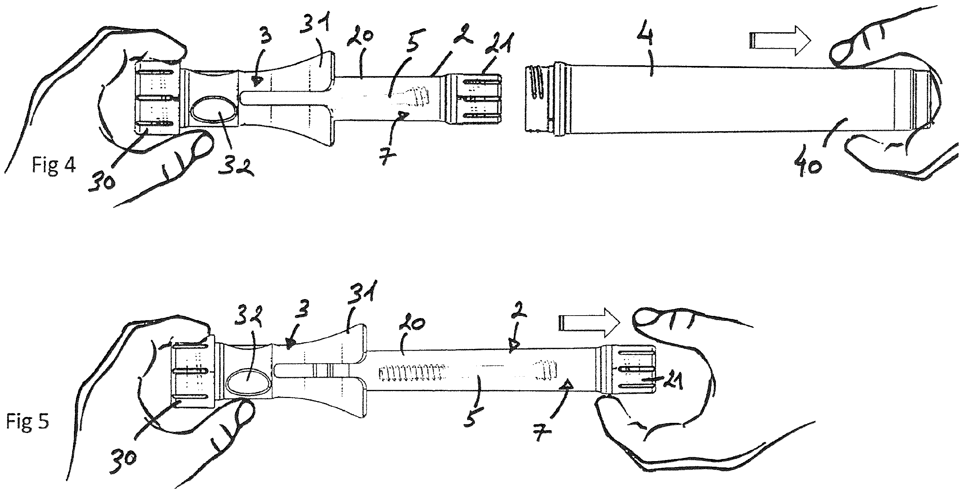

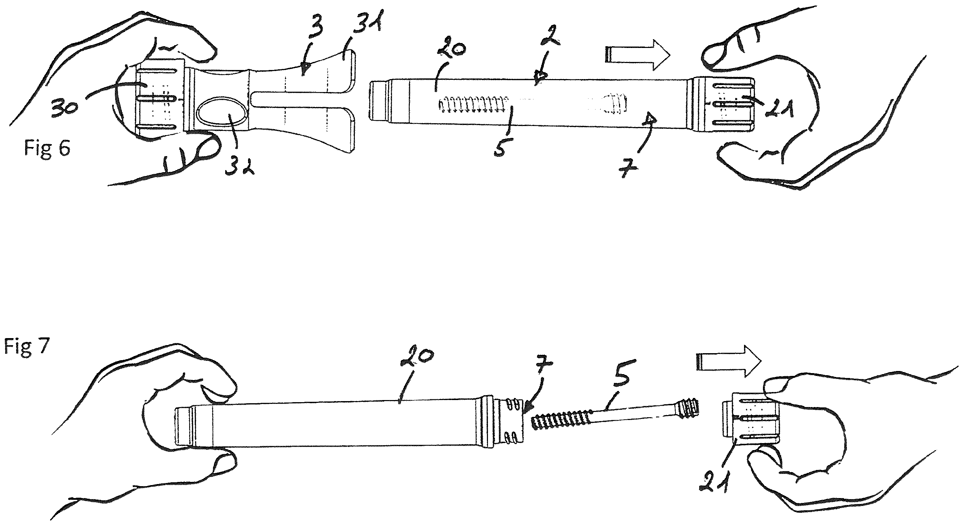

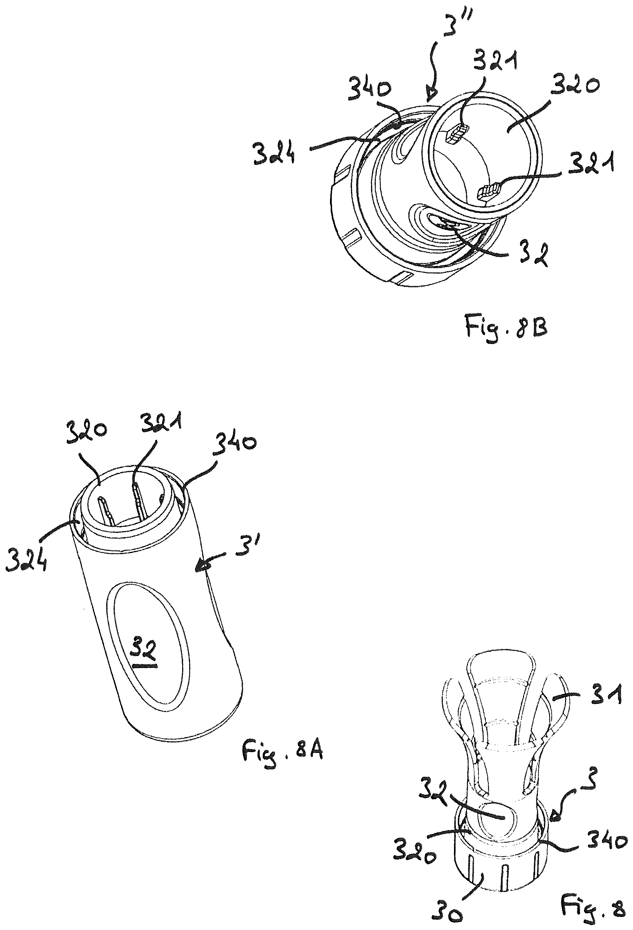

Ledit bouchon 3, et éventuellement la coiffe 4, présente(nt) au moins deux emplacements 32 prédéfinis permettant de positionner chacun au moins un doigt d'une personne pour lui permettre de saisir ledit bouchon ou ladite coiffe par pincement entre au moins deux doigts. Préférentiellement, au moins deux desdits emplacements 32 prédéfinis sont diamétralement opposés par rapport à l'axe de l'élément, bouchon 3 ou coiffe 4, sur lequel ils sont ménagés. Dans l'exemple illustré aux figures 1 à 8 et 8B, chaque emplacement 32 est ménagé sur une portion du bouchon destinée à être recouverte par la coiffe 4. Dans ce cas, ledit emplacement devient accessible une fois la coiffe retirée.

Selon un autre mode de réalisation du bouchon 3' illustré à la figure 8A, ledit emplacement 32 est ménagé sur une portion du bouchon 3' qui n'est pas destinée à être recouverte par la coiffe.

Avantageusement, le bouchon 3 ; 3' présente une longueur suffisante pour permettre à la personne de positionner ses doigts sur ledit bouchon, et en particulier de saisir le bouchon avec un pouce, sans risque de toucher l'élément support 2.

Lesdits éléments de support 2 et de protection 3, 4 de l'emballage s'emboitent à recouvrement partiel entre eux par friction et/ou par vissage. Les moyens de couplage des éléments les uns par rapport aux autres sont détaillés ci-après.

Dans l'exemple illustré aux figures, le bouchon 3 et la coiffe 4 s'emboitent entre eux par vissage, de même que les parties 20, 21 qui composent l'élément support 2. Préférentiellement, le filet et le taraudage ménagés sur les éléments qui s'emboitent sont configurés de manière à ne permettre le vissage desdits éléments entre eux qu'une seule fois. En particulier, on peut prévoir que le filetage et/ou le taraudage se déforment lors du vissage et du dévissage de sorte qu'un nouveau vissage des deux éléments entre eux n'est plus possible.

En variante, l'emboitement à recouvrement partiel desdits éléments de l'emballage pourrait être réalisé par simple friction, par exemple par pression ou serrage élastique. Un tel emboitement par simple friction permet de procéder au déboitement desdits éléments par simple traction de l'une ou de chacune des personnes sur un élément de l'emballage selon un geste naturel. On pourrait également envisager un emboîtement par clipsage.

Dans l'exemple illustré aux figures 1 à 8 et 8B, ledit bouchon 3 présente aussi au moins une partie 31, de préférence au moins une ailette, déformable, permettant d'emboiter partiellement ledit bouchon à l'intérieur de la coiffe. Ladite partie 31 est configurée pour, d'une part, à l'état recouvert de ladite partie 31 déformable du bouchon par la coiffe, venir en appui contre la face interne de coiffe 4 afin de permettre un couplage du bouchon à la coiffe par friction et, d'autre part, pour pouvoir se déformer afin de permettre la séparation de la coiffe 4 du bouchon 3.

Ledit bouchon 3 est formé par un corps creux ouvert à une extrémité et fermée à l'autre extrémité. La coiffe 4 est aussi formée par un corps creux ouvert à une extrémité et fermée à l'autre extrémité. L'extrémité fermée du bouchon 3 définie une partie de fond du bouchon. Comme détaillé ci-après, ledit bouchon 3 comprend des moyens de couplage avec l'élément support 2.

En particulier, ledit bouchon 3 comprend un corps sensiblement de révolution autour d'un axe correspondant à l'axe longitudinal dudit bouchon 3. Le corps du bouchon comprend une paroi périphérique 320 dite interne, et une paroi périphérique 340 dite externe qui entoure ladite paroi périphérique 320 interne.

La paroi périphérique 340 externe est sensiblement coaxiale à la paroi périphérique 320 interne.

Ladite paroi périphérique 340 externe entoure à écartement ladite paroi périphérique 320 interne pour définir entre lesdites parois périphériques un espace annulaire 324 d'insertion de la partie de la coiffe 4 destinée à être couplée audit bouchon 3. Ladite partie de la coiffe 4 destinée à être couplée audit bouchon 3 correspond à l'extrémité ouverte de la coiffe 4.

On peut prévoir de ménager sur les parois périphériques externe 340 et/ou interne 320 des moyens de vissage, tels que taraudage et/ou filetage, aptes à coopérer avec des moyens de vissage complémentaires, tels que filetage et/ou taraudage, ménagés sur la paroi périphérique de la coiffe 4.

En particulier, la face interne de la paroi périphérique 340 dite externe et/ou la face externe de la paroi périphérique 320 dite interne peu(ven)t présenter un taraudage et/ou un filetage pour coopérer avec un filet et/ou un taraudage complémentaire ménagé(s) sur la face périphérique externe et/ou la face périphérique interne du corps de la coiffe 4.

Dans l'exemple illustré aux figures, la face interne de la paroi périphérique 340 externe présente un taraudage apte à coopérer avec un filet ménagé sur la face externe de la paroi périphérique de la coiffe 4.

On peut également prévoir que l'écartement entre les parois périphériques externe 340 et interne 320 du bouchon soit configuré de telle sorte que l'emboitement de la coiffe 4 avec le bouchon 3 génère un pincement de la paroi périphérique de la coiffe 4 entre les parois périphériques externe 340 et interne 320 du bouchon.

Dans l'exemple illustré aux figures, le bouchon 3 et l'élément support 2 s'emboitent par enfoncement à force, c'est-à-dire par pression d'une partie d'extrémité de l'élément support 2 dans une partie creuse correspondante du bouchon 3.

Autrement dit, la face interne de la paroi périphérique interne 320 du bouchon 3 présente des moyens de maintien 321 permettant de maintenir la partie de l'élément support 2 emboitée à force à l'intérieur de l'espace délimité par la paroi périphérique interne 320 du bouchon 3.

Avantageusement, lesdits moyens de maintien 321 comprennent des ergots qui sont destinés à être en contact d'appui avec l'élément support 2 à l'état enfoncé dudit élément support dans le bouchon 3. On obtient ainsi un bon maintien de l'élément support 2 avec le bouchon 3 avant leur séparation en vue d'ouvrir ladite première chambre 7. En particulier, chaque ergot présente une partie formant rampe configurée de sorte que l'élément support 2 qui est emboité dans la paroi périphérique interne 320 pousse radialement les ergots vers l'extérieur et donc déforme la paroi périphérique interne 320 correspondante vers la paroi périphérique externe 340.

Plus précisément, comme illustré plus particulièrement aux figures 8A et 8B, les ergots 321 sont répartis sur la face périphérique interne du bouchon, autour de l'axe dudit bouchon 3, de préférence en étant espacés angulairement les uns des autres.

La présence desdits ergots à l'intérieur de la paroi périphérique interne 320 du bouchon permet de maintenir à force l'élément support 2 emboité dans le bouchon 3, tout en bénéficiant lors d'un tel emboitement de l'élément support 2 dans la paroi périphérique interne du bouchon, d'un rapprochement de la paroi périphérique interne 320 du bouchon par rapport à la paroi périphérique externe 340 dudit bouchon provoqué par appui de l'élément support 2 sur les ergots 321.

Le rapprochement entre elles par déformation élastique des parois périphériques interne 320 et externe 340 permet de renforcer l'étanchéité de la liaison au niveau de l'emboitement entre la coiffe 4 et le bouchon 3. En effet, les parois périphériques 320, 340 peuvent ainsi pincer la paroi périphérique de la partie du bouchon 3 emboitée dans l'espace ménagé entre lesdites parois périphériques interne 320 et externe 340 du bouchon.

Ainsi, lesdits moyens de maintien 321 ménagés à l'intérieur de la paroi périphérique interne 320 du bouchon 3 permettent d'améliorer non seulement le maintien de l'élément support 2 par rapport au bouchon 3, mais également l'étanchéité entre le bouchon 3 et la coiffe 4 dont la paroi périphérique est pincée entre lesdites parois périphériques interne 320 et externe 340 du bouchon 3.

Dans l'exemple illustré aux figures, le maintien entre la coiffe 4 et le bouchon 3 est assuré par les moyens de vissage complémentaires de type filetage et taraudage ménagés sur la coiffe 4 et le bouchon 3, et l'étanchéité entre la coiffe 4 et le bouchon 3 est obtenue par pincement de la paroi périphérique de la coiffe 4 entre lesdites parois périphériques 320 et 340 du bouchon 3 résultant de l'emboitement de l'élément support 2 dans le bouchon 3 qui déforme élastiquement la paroi périphérique 320 vers la paroi périphérique 340 du bouchon 3. Le maintien de l'élément support 2 dans le bouchon 3 est assuré par les ergots 321 qui enserrent l'élément support 2.

En outre, les ergots 321 forment des entretoises entre la paroi périphérique 320 et l'élément support 2 qui permettent de maintenir un écartement radial entre l'élément support 2 et la paroi périphérique 320. En outre, l'espacement entre les ergots 321 permet de définir un espace de communication de fluide entre l'intérieur de la paroi périphérique 320 et l'intérieur de la coiffe 4.

Un tel passage de communication entre l'intérieur de la coiffe 4 et l'intérieur de la paroi périphérique 320 permet à un gaz de stérilisation injecté dans la coiffe 4, par exemple via une membrane perméable au gaz, et de préférence imperméable aux bactéries, de se répandre à l'intérieur du volume délimité par la paroi périphérique 320 pour stériliser les deux chambres 7 et 8 de l'emballage, notamment lorsque l'élément support 2 est un élément ouvert ou lorsque l'extrémité de l'élément support 2 emboitée dans la paroi périphérique 320, présente une membrane perméable au gaz, et de préférence imperméable aux bactéries.

On peut prévoir que les moyens de maintien 321 de l'élément support 2 par rapport au bouchon 3 comprennent également des moyens de vissage, par exemple de type taraudage, aptes à coopérer avec des moyens de vissage complémentaires, par exemple de type filetage, ménagés sur la face externe de la paroi périphérique de l'élément support 2.

La combinaison de moyens de vissage et d'ergots permet ainsi de combiner un maintien de l'élément support 2 par rapport au bouchon 3 par vissage et également par friction en conservant les avantages de la liaison entre le bouchon 3 et la coiffe 4 rappelés ci-dessus.

En outre, dans l'exemple illustré aux figures, la paroi périphérique interne 320 s'étend en saillie de la paroi périphérique externe 340 du côté opposé au fond du bouchon 3.

Selon les variantes de réalisation de bouchon illustrées aux figures 8A et 8B, les bouchons 3' ; 3" présentent également chacun une partie creuse, munie d'ergots, dans laquelle est destinée à être enfoncée une partie d'extrémité de l'élément support correspondant.

L'élément support 2 et/ou le bouchon 3 et/ou la coiffe 4 présentent une partie, de préférence formée par une membrane, imperméable aux bactéries mais perméable aux gaz pour permettre une stérilisation gazeuse de ladite première chambre 7 et/ou de ladite deuxième chambre 8.

L'emballage décrit ci-dessus permet de mettre en oeuvre un procédé de déconditionnement qui comprend les étapes suivantes. Ce procédé est décrit en référence aux figures 1 à 8 pour lesquelles l'élément support est formé de deux pièces couplables/désaccouplables de manière à délimiter une cavité fermée qui renferme ledit objet. Comme illustré aux figures 1 à 4, une première personne, supposée en zone contaminée, sépare la coiffe 4 par rapport au bouchon 3 de manière à découvrir l'élément support 2 qui reste écarté des mains de la personne supposée contaminée grâce au fait que la main de ladite personne tient l'emballage restant par le bouchon 3, c'est-à-dire à écartement de l'élément support. La personne contaminée peut alors tendre à une deuxième personne, supposée en zone propre, l'emballage restant du côté de l'élément support 2. Ladite personne propre peut alors tirer sur l'élément support 2 pour entrainer la séparation dudit élément support 2 par rapport au bouchon 3 tenu par la première personne supposée contaminée. Ladite personne "propre" est par exemple le chirurgien au niveau du bloc opératoire.

Eventuellement, l'élément support 2 qui renferme l'objet 5 peut être mis dans une zone d'attente, en attente de l'utilisation dudit objet 5. Puis ladite deuxième personne ou une autre personne, supposée propre, peut ouvrir la première chambre 7 définie par ledit élément support 2 en séparant, ici par dévissage, les deux pièces 20, 21 qui forment ledit élément support pour saisir ladite pièce médicale.

Dans le cas non illustré aux figures, où l'élément support forme directement la première chambre qui loge ledit objet en coopération avec le bouchon, le procédé décrit ci-dessus est adapté en prévoyant que la personne contaminée tende à la deuxième personne, supposée en zone propre, l'emballage restant du côté de l'élément support 2, en orientant ledit emballage restant de telle sorte que l'élément support 2 se retrouve à une hauteur inférieure à celle du bouchon 3, de sorte que ledit objet 5 reste contenu dans l'élément support 2 lors de la séparation dudit élément support 2 par rapport au bouchon 3 pour éviter que ledit objet 5 ne tombe par terre.

Ainsi, la pièce médicale n'a été ni touchée ni lâchée au cours de son déconditionnement. En outre, la chambre contenant la pièce médicale n'est ouverte qu'en zone propre.

Grâce à la coiffe qui délimite une chambre de protection de la partie de saisie 20 de l'élément support 2, la personne propre touche une partie propre de l'emballage. En effet, les parties sales de l'emballage, à savoir le bouchon 3 et la coiffe 4, sont restées dans les mains de la personne sale. Ainsi, on est assuré que la main de la personne qui saisit l'élément support reste propre.

Bien entendu dans chacun des modes de réalisation on peut prévoir que la séparation de la coiffe et du bouchon soit réalisée par deux personnes différentes en zone sale au lieu d'une seule, l'une tenant une extrémité du bouchon et l'autre une extrémité de la coiffe.

Préférentiellement, les chambres 7 et 8 sont imperméables au moins aux bactéries. On peut prévoir qu'elles soient également imperméables à tout fluide. En variante, on peut prévoir que l'une et/ou l'autre, de préférence au moins la chambre de présentation 7 de l'objet, soit perméable uniquement aux gaz pour permettre une stérilisation gazeuse de la ou des chambres.

Ainsi, on peut prévoir, selon un mode de réalisation particulier de l'invention, qu'au moins une partie de la paroi du bouchon 3 qui délimite une partie de la chambre 7 de présentation de l'objet 5 est formée en matériau perméable au gaz, mais imperméable aux bactéries de manière à permettre une stérilisation gazeuse de la chambre de présentation et de l'objet, par exemple une pièce médicale, par exemple avec de l'oxyde d'éthylène ou par vapeur. Une telle conception de l'emballage permet de stériliser la ou les chambres autrement que par rayonnement.

On peut ainsi prévoir que le bouchon soit fermé à son extrémité, opposée à celle qui recouvre partiellement l'élément support, par une membrane adaptée à la stérilisation gazeuse.

La présente invention n'est nullement limitée aux modes de réalisation décrits et représentés, mais l'homme du métier saura y apporter toute variante conforme à son esprit.

Les modes de réalisation préférés sont définis dans les paragraphes suivants:
1. Ensemble médical, de préférence stérilisé, comprenant un objet (5) médical et un emballage (1) contenant ledit objet (5),
   ledit emballage comprenant un élément support (2) creux à l'intérieur duquel ledit objet (5) est libre,
   caractérisé en ce que ledit emballage comprend:
   - un premier élément de protection (3), appelé bouchon, couplable à l'élément support (2) dudit objet (5), de préférence par emboîtement à recouvrement partiel, de telle sorte que l'élément support (2) présente une partie (20) de saisie qui s'étend en saillie dudit bouchon (3),
   ledit élément support (2) délimitant à lui seul ou en coopération avec le bouchon (3) une première chambre (7) renfermant ledit objet (5),
   - un deuxième élément de protection (4) creux, appelé coiffe,
   ladite coiffe (4) étant couplable au bouchon (3), de préférence par emboîtement à recouvrement partiel, de manière à délimiter en coopération avec ledit bouchon (3) une deuxième chambre (8) à l'intérieur de laquelle s'étend la partie de saisie (20) de l'élément support (2),
   et en ce que, à l'état couplé de la coiffe (4) au bouchon (3), une partie du bouchon (3) s'étend en saillie de la coiffe (4).
2. Ensemble médical selon le paragraphe 1, caractérisé en ce que ledit bouchon (3) comprend une paroi périphérique (320) dite interne, et une paroi périphérique (340) dite externe qui entoure à écartement ladite paroi périphérique (320) interne pour définir entre lesdites parois périphériques (320, 340) un espace annulaire (324) d'emboîtement de la partie de la coiffe (4) destinée à être couplée audit bouchon (3), la paroi périphérique (320) interne définissant un espace d'emboîtement de la partie de l'élément support (2) destinée à être couplée audit bouchon (3).
3. Ensemble médical selon le paragraphe 2, caractérisé en ce que la face interne de la paroi périphérique interne (320) du bouchon (3) est munie de moyens de maintien (321) permettant de maintenir la partie de l'élément support (2) emboîtée à l'intérieur de l'espace délimité par ladite paroi périphérique interne (320) du bouchon (3).
4. Ensemble médical selon le paragraphe 3, caractérisé en ce que lesdits moyens de maintien (321) comprennent des ergots qui sont destinés à être en contact d'appui avec l'élément support (2) à l'état emboîté dudit élément support (2) dans le bouchon (3),
   et en ce que les ergots (321) sont répartis sur la face périphérique interne (320) du bouchon (3), autour de l'axe dudit bouchon (3), en étant espacés angulairement les uns des autres.
5. Ensemble médical selon le paragraphe 4, caractérisé en ce que chaque ergot (321) comprend une partie formant rampe, dirigée vers l'axe du bouchon (3) en partant d'un point bas de la rampe situé du côté de l'extrémité ouverte du bouchon (3) vers un point haut de ladite rampe situé du côté de l'extrémité fermée dudit bouchon (3), de sorte que, lors de l'emboitement de l'élément support (2) à l'intérieur de l'espace défini par la paroi périphérique interne (320) du bouchon (3), ledit élément support (2), par appui sur ladite rampe, pousse radialement ledit ergot (321) et déforme la paroi périphérique interne (320) correspondante vers la paroi périphérique externe (340).
6. Ensemble médical selon le paragraphe 4 ou 5, caractérisé en ce que les ergots (321) forment des entretoises permettant de maintenir un écartement radial entre l'élément support (2) et la paroi périphérique interne (320) du bouchon (3), l'espacement entre les ergots (321) définissant un espace de communication de fluide entre l'intérieur de la paroi périphérique (320) du bouchon (3) et l'intérieur de la coiffe (4).
7. Ensemble médical selon l'un des paragraphes 2 à 6, caractérisé en ce que lesdites parois périphériques externe (340) et/ou interne (320) du bouchon (3) comprennent des moyens de vissage aptes à coopérer avec des moyens de vissage complémentaires ménagés sur la coiffe (4).
8. Ensemble médical selon le paragraphe 7, caractérisé en ce que la face interne de la paroi périphérique (340) du bouchon (3) externe présente un taraudage apte à coopérer avec un filet ménagé sur la face externe de la paroi périphérique de la coiffe (4).
9. Ensemble médical selon l'un des paragraphes précédents, caractérisé en ce que, ledit élément support (2) délimitant à lui seul ladite première chambre (7) renfermant ledit objet (5), ledit élément support (2) est formé d'au moins deux pièces (20, 21) couplables/désaccouplables l'une par rapport à l'autre, de préférence par emboîtement à recouvrement partiel, pour former ladite première chambre renfermant ledit objet (5).
10. Ensemble médical selon l'un des paragraphes 1 à 8, caractérisé en ce que, ledit élément support (2) délimitant en coopération avec le bouchon (3) ladite première chambre (7) renfermant ledit objet (5), ledit élément support (2) délimite une cavité ouverte destinée à être refermée par ledit bouchon (3).
11. Ensemble médical selon l'un des paragraphes précédents, caractérisé en ce que ledit bouchon (3), et éventuellement la coiffe (4), présente(nt) des emplacements prédéfinis (32) permettant de positionner au moins deux doigts, de préférence trois doigts, d'une personne pour lui permettre de saisir ledit bouchon, et éventuellement ladite coiffe, au niveau desdits emplacements prédéfinis.
12. Ensemble médical selon l'un des paragraphes précédents, caractérisé en ce que l'élément support (2) et/ou le bouchon (3) et/ou la coiffe (4) présente(nt) une partie, de préférence formée par une membrane, imperméable aux bactéries mais perméable aux gaz pour permettre une stérilisation gazeuse de ladite première chambre (7) et/ou de ladite deuxième chambre (8).
13. Procédé de déballage d'un objet médical contenu dans un emballage (1) d'ensemble médical, de préférence stérilisé, conforme à l'une des paragraphes précédents, caractérisé en ce que ledit procédé comprend les étapes suivantes:
   a) - séparation de la coiffe (4) par rapport au bouchon (3) par une première personne de manière à découvrir l'élément support (2),
   b) - saisie par une deuxième personne de l'élément support (2),
   c) - séparation dudit élément support (2) par rapport au bouchon (3) tenu par la première personne.
14. Procédé de déballage selon le paragraphe 13, ledit emballage étant conforme au paragraphe 9, caractérisé en ce que ledit procédé comprend les étapes supplémentaires suivantes :
   d) - éventuellement, mise en attente dudit élément support (2) qui renferme l'objet (5) dans une zone d'attente, en attente de l'utilisation dudit objet (5),
   e) - ouverture de la première chambre (7) définie par ledit élément support (2) pour en extraire ledit objet (5).
15. Procédé de déballage selon le paragraphe 13, ledit emballage étant conforme au paragraphe 10, caractérisé en ce que ledit procédé comprend entre les étapes a) et b) l'étape supplémentaire suivante de présentation, par la première personne à la deuxième personne, de l'emballage restant, formé du bouchon (3) et de l'élément support (2), en lui présentant l'extrémité dudit emballage restant formée par ledit élément support (2), en orientant ledit emballage restant de telle sorte que l'élément support (2) se retrouve à une hauteur inférieure à celle du bouchon (3) de sorte que ledit objet (5) reste contenu dans l'élément support (2) lors de la séparation dudit élément support (2) par rapport au bouchon (3) pour éviter que ledit objet (5) ne tombe par terre.

## Revendications

1. Emballage médical contenant un objet médical, comprenant :
un élément de support creux à l'intérieur duquel l'objet est libre ;
un bouchon qui est configuré de manière à ce qu'il soit couplé à l'élément de support creux de l'objet de telle sorte que l'élément de support creux présente une partie de saisie qui s'étend en saillie du bouchon, la partie de saisie de l'élément de support creux étant configurée de manière à ce qu'elle soit tenue en main par une personne ;
une première chambre qui est configurée de manière à ce qu'elle renferme l'objet, dans lequel la première chambre est délimitée par l'élément de support creux à lui seul ou en coopération avec le bouchon ;
une coiffe de protection creuse qui est configurée de manière à ce qu'elle soit couplée au bouchon de telle sorte qu'elle délimite, en coopération avec le bouchon, une seconde chambre à l'intérieur de laquelle la partie de saisie de l'élément de support creux s'étend, la coiffe de protection creuse étant configurée de manière à ce qu'elle soit séparée du bouchon ; dans lequel :
lorsque la coiffe de protection creuse est couplée au bouchon, une partie du bouchon s'étend en saillie de la coiffe de protection creuse, et est configurée de manière à ce qu'elle forme une extrémité de saisie du bouchon qui est configurée de manière à ce qu'elle soit tenue en main par une personne, lorsque la coiffe de protection creuse est séparée du bouchon ; dans lequel :
une partie de l'élément de support creux est configurée de manière à ce qu'elle soit emboîtée en force à l'intérieur du bouchon, et, lorsque la coiffe de protection creuse est séparée du bouchon, l'élément de support creux reste couplé au bouchon tandis que l'élément de support creux peut également être séparé du bouchon lorsqu'une personne tient la partie de saisie de l'élément de support creux et exerce une traction sur l'élément de support creux de manière à le séparer du bouchon.

2. Emballage médical selon la revendication 1, dans lequel le bouchon comprend une paroi périphérique interne et une paroi périphérique externe qui entoure à écartement la paroi périphérique interne de manière à définir, entre les parois périphériques interne et externe, un espace annulaire pour l'emboîtement de la partie de la coiffe de protection creuse qui est configurée de manière à ce qu'elle soit couplée au bouchon, la paroi périphérique interne définissant un espace pour l'emboîtement de la partie de l'élément de support creux qui est configurée de manière à ce qu'elle soit couplée au bouchon.

3. Emballage médical selon la revendication 2, dans lequel une face interne de la paroi périphérique interne du bouchon est munie de moyens de maintien pour maintenir la partie de l'élément de support creux qui est emboîtée à l'intérieur de l'espace qui est délimité par la paroi périphérique interne du bouchon.

4. Emballage médical selon la revendication 3, dans lequel les moyens de maintien comprennent des ergots qui sont configurés de manière à ce qu'ils soient en contact d'appui avec l'élément de support creux dans l'état dans lequel l'élément de support creux est emboîté dans le bouchon, et dans lequel les ergots sont répartis sur la face périphérique interne du bouchon, autour de l'axe du bouchon, en étant espacés angulairement les uns des autres.

5. Emballage médical selon la revendication 4, dans lequel chaque ergot comprend une partie formant rampe, dirigée vers l'axe du bouchon en partant d'un point bas de la rampe qui est situé du côté de l'extrémité ouverte du bouchon vers un point haut de la rampe qui est situé du côté de l'extrémité fermée du bouchon de telle sorte que, lorsque l'élément de support creux est emboîté à l'intérieur de l'espace qui est défini par la paroi périphérique interne du bouchon, l'élément de support creux, par appui sur la rampe, pousse radialement l'ergot et déforme la paroi périphérique interne correspondante vers la paroi périphérique externe.

6. Emballage médical selon la revendication 2, dans lequel au moins l'une des parois périphériques externe et interne du bouchon comprend des moyens de vissage aptes à coopérer avec des moyens de vissage complémentaires ménagés sur la coiffe de protection creuse.

7. Emballage médical selon la revendication 6, dans lequel la face interne de la paroi périphérique externe du bouchon présente un taraudage apte à coopérer avec un filet qui est ménagé sur la face externe d'une paroi périphérique de la coiffe de protection creuse.

8. Emballage médical selon la revendication 1, dans lequel, lorsque l'élément de support creux délimite à lui seul la première chambre qui renferme l'objet, l'élément de support creux est formé d'au moins deux pièces qui sont configurées de manière à ce qu'elles soient couplées/découplées l'une par rapport à l'autre pour former la première chambre qui renferme l'objet.

9. Emballage médical selon la revendication 1, dans lequel, lorsque l'élément de support creux délimite, en coopération avec le bouchon, la première chambre qui renferme l'objet, l'élément de support creux délimite une cavité ouverte qui est configurée de manière à ce qu'elle soit fermée par le bouchon.

10. Emballage médical selon la revendication 1, dans lequel au moins un élément constitutif pris parmi l'élément de support creux, le bouchon et la coiffe de protection creuse comporte une partie qui est imperméable aux bactéries mais perméable aux gaz pour permettre une stérilisation gazeuse d'au moins une chambre prise parmi la première chambre et la seconde chambre.

11. Emballage médical selon la revendication 1, dans lequel le bouchon peut être couplé à l'élément de support creux de l'objet par emboîtement à recouvrement partiel.

12. Emballage médical selon la revendication 1, dans lequel la coiffe de protection creuse est apte à être couplée au bouchon par emboîtement à recouvrement partiel.

13. Procédé de déballage d'un objet médical contenu dans un emballage médical d'un ensemble médical tel que revendiqué selon la revendication 1, dans lequel le procédé comprend les étapes suivantes :
la séparation de la coiffe de protection creuse par rapport au bouchon par une première personne de manière à découvrir l'élément de support creux ;
la saisie de l'élément de support creux par une seconde personne ; et
la séparation de l'élément de support creux par rapport au bouchon qui est tenu par la première personne.

14. Procédé de déballage tel que revendiqué selon la revendication 13, dans lequel le procédé comprend l'étape supplémentaire suivante d'ouverture de la première chambre qui est définie par l'élément de support creux pour en extraire l'objet.

15. Procédé de déballage tel que revendiqué selon la revendication 14, dans lequel le procédé comprend, entre l'étape de séparation et l'étape de saisie, l'étape supplémentaire suivante selon laquelle la première personne propose à la seconde personne l'emballage restant qui est formé du bouchon et de l'élément de support creux en lui présentant l'extrémité de l'emballage restant qui est formée par l'élément de support creux, en orientant l'emballage restant de telle sorte que l'élément de support creux soit à une hauteur inférieure à celle du bouchon, de telle sorte que l'objet reste contenu dans l'élément de support creux pendant la séparation de l'élément de support creux par rapport au bouchon, de manière à éviter que l'objet ne tombe par terre.
